# EUROPÄISCHE PATENTANMELDUNG

(11) **EP 0 780 377 A1**
(43) Veröffentlichungstag der Anmeldung: **25.06.1997**
(21) Anmeldenummer: 96116478.7
(22) Anmeldetag: 15.10.1996
(51) Int. Cl.: C07D 229/00, C08G 18/79

(54) **Verfahren zur Herstellung eines isocyanurathaltigen Uretdions aus Isophorondiisocyanat sowie das danach hergestellte Uretdion**

(30) Priorität: 22.12.1995 DE 19548250
(71) Anmelder: HÜLS AKTIENGESELLSCHAFT, 45764 Marl (DE)
(72) Erfinder: Wolf, Elmar, Dr., 45661 Recklinghausen (DE)

(57) **Zusammenfassung**

Farbloses Uretdion aus Isophorondiisocyanat, das in der Hitze zu mindestens 90 % wieder in Isophorondiisocyanat rückspaltbar und zur gezielten Kettenverlängerung mit Diolen geeignet ist, erhältlich dadurch, daß man Isophorondiisocyanat ohne oder in einem inerten Lösemittel in Gegenwart des folgenden Katalysatorgemisches A und B: 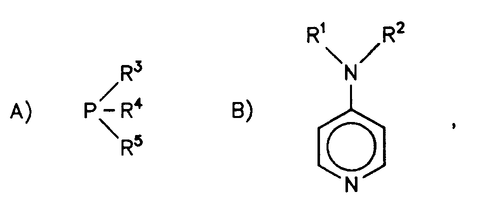 bei Temperaturen von 0 - 60 °C mit 0,2 - 4 Gew.-% der Katalysatoren A + B umsetzt, und das Reaktionsprodukt nach einem Umsatz von 10 - 70 % ohne vorherige Desaktivierung des Katalysatorgemisches aus dem Reaktionsgemisch durch Dünnschichtdestillation als Rückstand isoliert, wobei R¹ und R² gleiche oder verschiedene Alkylreste mit 1 bis 8 C-Atomen darstellen oder mit dem am Ring gebundenen N-Atom einen gemeinsamen 5- oder 6-Ring bilden können, der anstelle einer CH₂-Gruppe eine CH-CH₃-Gruppe, eine N-CH₃-Gruppe oder ein O-Atom enthalten kann, R³, R⁴, R⁵ gleiche oder verschiedene (Cyclo)alkylreste mit 4 - 12 C-Atomen bedeuten.

## Beschreibung

Die vorliegende Erfindung betrifft ein Verfahren zur Herstellung eines isocyanuratgruppenhaltigen Uretdions, das aus Isophorondiisocyanat (abgekürzt: IPDI) hergestellt wird.

Die Dimerisierung von aliphatischen Diisocyanaten wird erstmals in der DE-OS 16 70 720 beschrieben. Als Katalysatoren werden tert. Phosphine eingesetzt. Die gemäß der Lehre der DE-OS 16 70 720 aus Isocyanaten hergestellten aliphatischen Uretdione enthalten allerdings in beträchtlichem Maße die entsprechenden Isocyanurate als Verunreinigung (Beispiel 1 ca. 40 % Butylisocyanurat; Beispiel 2a 50 % Ethylisocyanurat). Auch bei der Dimerisierung des IPDI entsprechend dem in der DE-OS 16 70 720 beschriebenen Verfahren wird kein reines Uretdion, sondern nur ein Gemisch von Reaktionsprodukten erhalten, das maximal aus 80 % IPDI-Uretdion besteht; der Rest ist nicht abtrennbares IPDI-Isocyanurat.

Die DE-OS 19 34 763 befaßt sich ausschließlich mit der Oligomerisierung von IPDI mit tertiären Phosphinen. Die gemäß der Lehre dieser Literatur erhaltenen Reaktionsprodukte bestehen aus ca. 60 Gewichtsteile dimerem (in der Hitze spaltbarem) und ca. 40 Gewichtsteile trimerem bzw. höheroligomerisiertem (in der Hitze nicht mehr spaltbarem) IPDI. Durch geeignete Verfahrensvarianten (z. B. niedriger Umsatz, niedrige Temperatur) kann der Dimeranteil noch auf ca. 80 Gewichtsteile erhöht werden. Eine weitere Erhöhung des Uretdiongehaltes ist nicht mehr möglich, da der Katalysator (tert. Phosphine) nicht nur die Dimerisierung, sondern auch die Trimerisierung des IPDI zum entsprechenden Isocyanurat katalysiert. Ein solches isocyanurathaltiges Uretdion des IPDI ist zur gezielten Kettenverlängerung mit Diolen zur Herstellung von wertvollen Ausgangsverbindungen für die Polyurethanchemie (z. B. blockierungsmittelfreie PUR-Pulverhärter) nicht geeignet, da bereits bei der Herstellung eines solchen kettenverlängerten isocyanurathaltigen Uretdions des IPDI mit zumindestens teilweiser Gelierung zu rechnen ist.

In der DE-OS 37 39 549 wird ein IPDI-Uretdion beschrieben, das isocyanuratfrei ist und somit zur Herstellung von blockierungsmittelfreien PUR-Pulverhärtern durch Reaktion mit Diolen sehr gut geeignet ist. Als Katalysatoren für die Dimerisierung von IPDI werden 4-N-dialkylsubstituierte Pyridine, speziell 4-Dimethylaminopyridin (DMAP), eingesetzt. Nachteilig bei dem nach der DE-OS 37 39 549 hergestellten Uretdion des IPDI ist seine mehr oder weniger intensiv gelbe Farbe, die natürlich auch bei dessen Reaktionsprodukten mit Diolen vorhanden ist.

Überraschenderweise wurde ein nahezu farbloses IPDI-Uretdion erhalten, das höchstens 10 Gew.-% Trimeres, in der Hitze nicht mehr spaltbares, IPDI enthält und zur Kettenverlängerung mit Diolen geeignet ist, wenn als Dimerisierungskatalysator ein Gemisch aus tert. Phosphinen und 4-N-dialkylsubstituierten Pyridinen eingesetzt wird.

Gegenstand der vorliegenden Erfindung ist daher ein farbloses Uretdion aus Isophorondiisocyanat, das in der Hitze zu mindestens 90 % wieder in Isophorondiisocyanat rückspaltbar und zur gezielten Kettenverlängerung mit Diolen geeignet ist, erhältlich dadurch, daß man Isophorondiisocyanat ohne oder in einem inerten Lösemittel in Gegenwart des folgenden Katalysatorgemisches A und B: 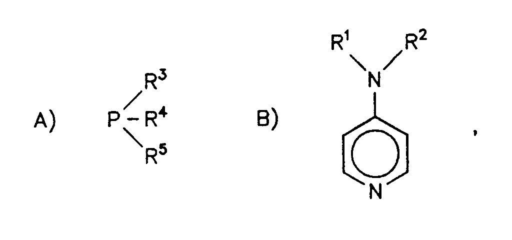 bei Temperaturen von 0 - 60 °C mit 0,2 - 4 Gew.-% der Katalysatoren A + B umsetzt und das Reaktionsprodukt nach einem Umsatz von 10 - 70 % ohne vorherige Desaktivierung des Katalysatorgemisches aus dem Reaktionsgemisch durch Dünnschichtdestillation als Rückstand isoliert, wobei R¹ und R² gleiche oder verschiedene Alkylreste mit 1 bis 8 C-Atomen darstellen oder mit dem am Ring gebundenen N-Atom einen gemeinsamen 5- oder 6-Ring bilden können, der anstelle einer CH₂-Gruppe eine CH-CH₃-Gruppe, eine N-CH₃-Gruppe oder ein O-Atom enthalten kann, R³, R⁴, R⁵ gleiche oder verschiedene (Cyclo)alkylreste mit 4 - 12 C-Atomen bedeuten.

Gegenstand der Erfindung ist ebenfalls ein Verfahren zur Herstellung eines Uretdions aus Isophorondiisocyanat.

Die Katalysatoren A + B werden in Mengen von 0,2 - 4 Gew. -%, vorzugsweise 0,5 - 2 Gew.-%, eingesetzt. Bei den Katalysatoren A handelt es sich um Trialkylphosphine, wie z. B. Tributyl-, Trihexyl-, Trioctyl-, Tridodecylphosphin. Die Katalysatoren B des Katalysatorgemisches sind N,N-disubstituierte 4-Aminopyridin-Derivate, wie z. B. 4-Dimethylaminopyridin, 4-Diethylaminopyridin, 4-Pyrrolidinopyridin, 4-Piperidinopyridin und 4-(4-Methylpiperidino)-pyridin.

Die Herstellung des erfindungsgemäßen Uretdions aus Isophorondiisocyanat erfolgt derart, daß zunächst IPDI mit Hilfe des beschriebenen Katalysatorgemisches A + B bis zu einem Umsatz, der die Förderung des Reaktionsgemisches im flüssigen Zustand (bei Raumtemperatur) noch zuläßt, bevorzugt 40 - 60 % IPDI-Umsatz, umgesetzt wird, und danach das nicht umgesetzte IPDI mit dem Katalysatorgemisch durch Dünnschichtdestillation vom Reaktionsprodukt abgetrennt wird. Das abdestillierte IPDI sowie die Katalysatoren A + B können wieder zur Reaktion eingesetzt werden. Die Reaktionstemperatur liegt in einem Bereich von 0 bis 60 °C, bevorzugt 10 bis 30 °C. Bei höheren Temperaturen macht sich deutlich die katalytische Umwandlung des Uretdions in das thermodynamisch stabilere Isocyanurat bemerkbar.

Die Reaktionszeit, die Zeit, in der z. B. 40 - 60 % des IPDI umgesetzt sind, hängt, bei konstanter Temperatur, in hohem Maß von der Konzentration sowie von der Art der eingesetzten Katalysatoren ab. Sie beträgt in der Regel 10 - 90 h. Die Reaktion kann in polaren Lösungsmitteln, wie Estern, Ethern und Ketonen, oder lösungsmittelfrei durchgeführt werden. Bevorzugt arbeitet man lösungsmittelfrei.

Wird zur Dimerisierung des IPDI nur der Katalysator A (Trialkylphosphine) eingesetzt, so erhält man nahezu farblose Reaktionsprodukte, die, wie bereits eingangs erwähnt, neben dem gewünschten IPDI-Uretdion noch ca. 25 Gew.-% des trimeren IPDI (Isocyanurat des IPDI) enthalten und somit als Ausgangsverbindungen zur Herstellung von blockierungsmittelfreien PUR-Pulvern, die zunehmend an wirtschaftlicher Bedeutung gewinnen, nicht in Frage kommen. Werden zur Dimerisierung von IPDI nur die Verbindungen B (4-Dimethylaminopyridin) eingesetzt, erhält man ein isocyanuratgruppenfreies IPDI-Uretdion und somit eine zur Herstellung von blockierungsmittelfreien PUR-Pulvern geeignete Ausgangsverbindung, die jedoch den Nachteil der intensiven gelben Farbe aufweist.

Es war überraschend, daß durch Kombination der beiden Katalysatoren ein Katalysatorgemisch für die Dimerisierung des IPDI erhalten wurde, das die vorteilhaften Eigenschaften der einzelnen Katalysatoren A + B aufweist, ohne mit deren Nachteilen behaftet zu sein, wenn man sie einzeln verwendet.

Die nach dem erfindungsgemäßen Verfahren hergestellten Uretdione zeichnen sich gegenüber den Uretdionen der DE-OS 37 39 549 durch eine deutlich verbesserte Farbqualität aus.

### Beispiel 1:

IPDI wurde mit:
a) 0,5 Gew.-% Trioctylphosphin (Vergleichsbeispiel)
b) 0,5 Gew.-% DMAP (Vergleichsbeispiel)
c) 0,5 Gew.-% Trioctylphosphin + 0,5 Gew.-% DMAP
d) 1,0 Gew.-% DMAP (Vergleichsbeispiel)
gemischt und bei Raumtemperatur stehengelassen. In Abhängigkeit von der Zeit wurde der Reaktionsverlauf durch Bestimmung des Brechungsindex sowie des NCO-Gehaltes verfolgt. Gleichzeitig wurde mit fortschreitender Reaktion die Farbe (Farbzahl nach Hazen) gemessen. Nach 3 d Reaktionszeit wurde das Reaktionsprodukt durch Dünnschichtdestillation bei 120 °C/0,1 mbar aus dem Reaktionsgemisch isoliert.

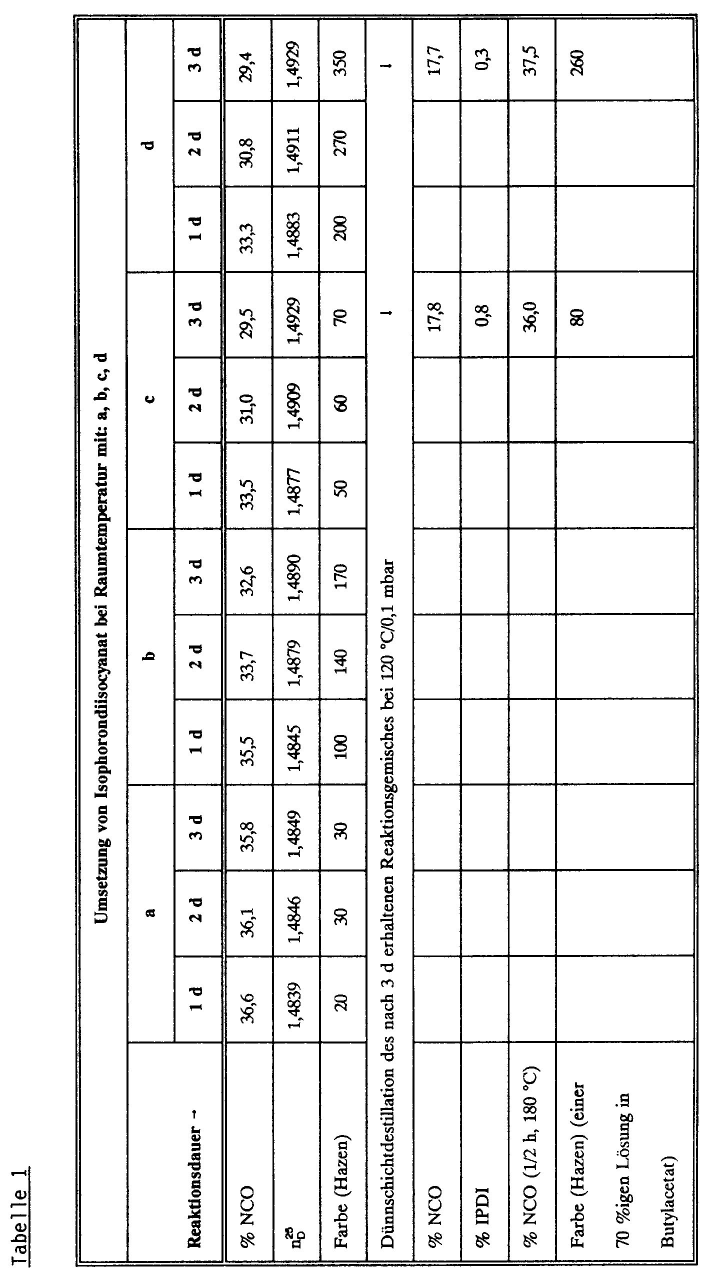

### Beispiel 2:

Isophorondiisocyanat wurde mit:
a) 1 Gew.-% Trioctylphosphin (Vergleichsbeispiel)
b) 1 Gew.-% DMAP (Vergleichsbeispiel)
c) 1 Gew.-% DMAP + 0,5 Gew.-% Trioctylphosphin
entsprechend den im Beispiel 1 angegebenen Reaktionsbedingungen umgesetzt.

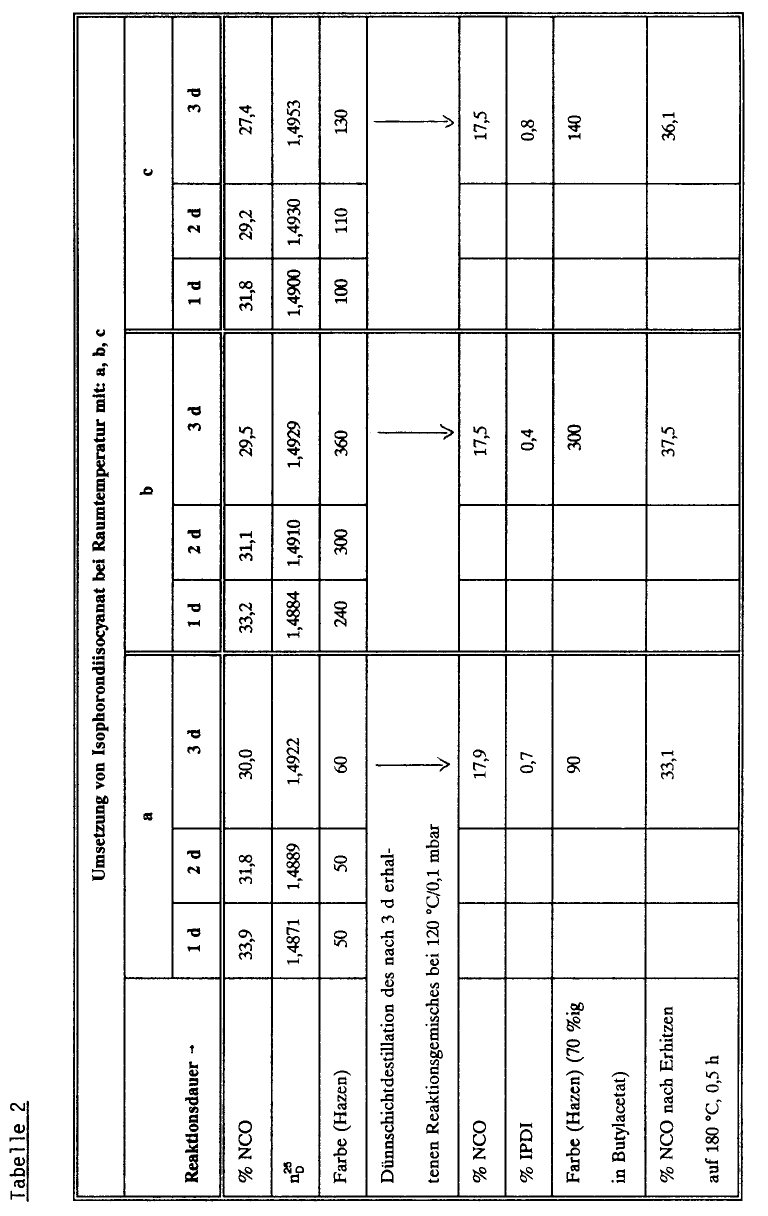

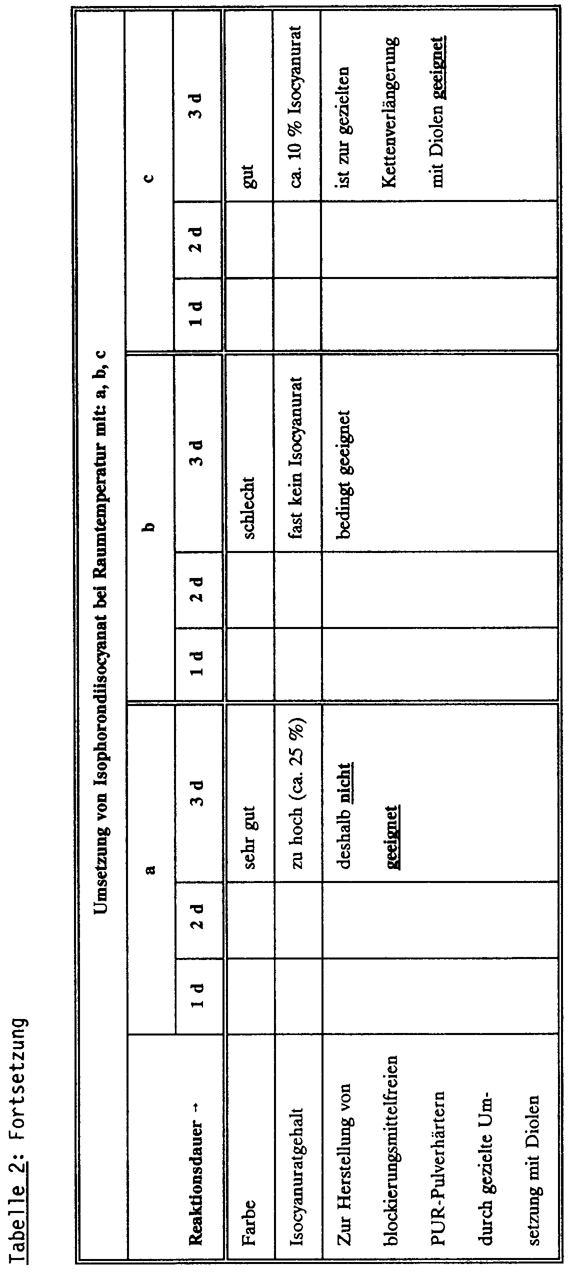

## Patentansprüche

1. Farbloses Uretdion aus Isophorondiisocyanat, das in der Hitze zu mindestens 90 % wieder in Isophorondiisocyanat rückspaltbar und zur gezielten Kettenverlängerung mit Diolen geeignet ist, erhältlich dadurch, daß man Isophorondiisocyanat ohne oder in einem inerten Lösemittel in Gegenwart des folgenden Katalysatorgemisches A und B: 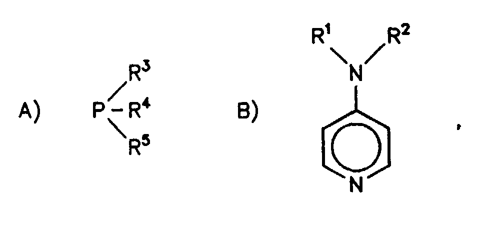 bei Temperaturen von 0 - 60 °C mit 0,2 - 4 Gew.-% der Katalysatoren A + B umsetzt und das Reaktionsprodukt nach einem Umsatz von 10 - 70 % ohne vorherige Desaktivierung des Katalysatorgemisches aus dem Reaktionsgemisch durch Dünnschichtdestillation als Rückstand isoliert, wobei R¹ und R² gleiche oder verschiedene Alkylreste mit 1 bis 8 C-Atomen darstellen oder mit dem am Ring gebundenen N-Atom einen gemeinsamen 5- oder 6-Ring bilden können, der anstelle einer CH₂-Gruppe eine CH-CH₃-Gruppe, eine N-CH₃-Gruppe oder ein O-Atom enthalten kann, R³, R⁴, R⁵ gleiche oder verschiedene (Cyclo)alkylreste mit 4 - 12 C-Atomen bedeuten.

2. Uretdion aus Isophorondiisocyanat nach Anspruch 1,
dadurch gekennzeichnet,
daß die Umsetzung bei Temperaturen von 10 bis 30 °C erfolgt.

3. Uretdion aus Isophorondiisocyanat nach den Ansprüchen 1 bis 2,
dadurch gekennzeichnet,
daß das Reaktionsgemisch nach einem Umsatz von 20 bis 50 % isoliert wird.

4. Uretdion aus Isophorondiisocyanat nach den Ansprüchen 1 bis 3,
dadurch gekennzeichnet,
daß 0,5 bis 2 Gew.-% der Katalysatoren A + B eingesetzt werden.

5. Uretdion aus Isophorondiisocyanat nach den Ansprüchen 1 bis 4,
dadurch gekennzeichnet,
daß als Katalysator A Trialkylphosphine, ausgewählt aus der Gruppe Tributyl-, Trihexyl-, Trioctyl-, Tridodecylphosphin, eingesetzt werden.

6. Uretdion aus Isophorondiisocyanat nach den Ansprüchen 1 bis 5,
dadurch gekennzeichnet,
daß als Katalysator B 4-Dimethylaminopyridin, 4-Diethylaminopyridin, 4-Pyrrolidinopyridin, 4-Piperidinopyridin und 4-(4-Methylpiperidino)-pyridin eingesetzt werden.

7. Uretdion aus Isophorondiisocyanat nach den Ansprüchen 1 bis 6,
dadurch gekennzeichnet,
daß lösemittelfrei umgesetzt wird.

8. Verfahren zur Herstellung von farblosem Uretdion aus Isophorondiisocyanat, das in der Hitze zu mindestens 90 % wieder in Isophorondiisocyanat rückspaltbar und zur gezielten Kettenverlängerung mit Diolen geeignet ist, dadurch gekennzeichnet, daß man Isophorondiisocyanat ohne oder in einem inerten Lösemittel in Gegenwart des folgenden Katalysatorgemisches A und B: 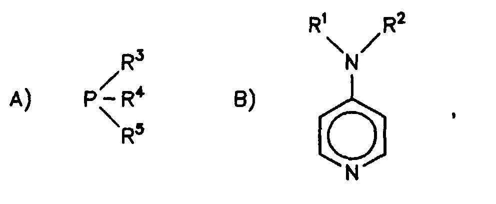 bei Temperaturen von 0 - 60 °C mit 0,2 - 4 Gew.-% der Katalysatoren A + B umsetzt, und das Reaktionsprodukt nach einem Umsatz von 10 - 70 % ohne vorherige Desaktivierung des Katalysatorgemisches aus dem Reaktionsgemisch durch Dünnschichtdestillation als Rückstand isoliert, wobei R¹ und R² gleiche oder verschiedene Alkylreste mit 1 bis 8 C-Atomen darstellen oder mit dem am Ring gebundenen N-Atom einen gemeinsamen 5- oder 6-Ring bilden können, der anstelle einer CH₂-Gruppe eine CH-CH₃-Gruppe, eine N-CH₃-Gruppe oder ein O-Atom enthalten kann, R³, R⁴, R⁵ gleiche oder verschiedene (Cyclo)alkylreste mit 4 - 12 C-Atomen bedeuten.
